# EUROPÄISCHE PATENTSCHRIFT

(11) **EP 3 096 827 B1**
(45) Veröffentlichungstag und Bekanntmachung des Hinweises auf die Patenterteilung: **21.08.2019**
(21) Anmeldenummer: 14835696.7
(22) Anmeldetag: 12.12.2014
(51) Int. Cl.: A61M 16/12, A61M 16/10

(54) **VORRICHTUNG ZUR BEATMUNG UND VERFAHREN ZUR ANREICHERUNG VON ATEMGAS MIT ADDITIVEN**
VENTILATION DEVICE AND METHOD FOR ENRICHING BREATHING GAS WITH ADDITIVES
DISPOSITIF DE VENTILATION ET PROCÉDÉ D'ENRICHISSEMENT DE GAZ RESPIRATOIRES PAR DES ADDITIFS

(30) Priorität: 23.01.2014 DE 102014000884
(43) Veröffentlichungstag der Anmeldung: 30.11.2016
(73) Patentinhaber: Weinmann Emergency Medical Technology GmbH + Co. KG, 22525 Hamburg (DE)
(72) Erfinder: DIEHL, Marcus, 22587 Hamburg (DE); WOLTMANN, Rene, 22846 Norderstedt (DE); JACOBSON, Alexander, 25479 Ellerau (DE)
(74) Vertreter: Klickow & Wetzel PartGmbB
(86) Internationale Anmeldenummer: PCT/DE2014/000642
(87) Internationale Veröffentlichungsnummer: WO 2015/110098

(56) Entgegenhaltungen:
- DE-A1-102004 045 693
- DE-A1-102007 058 807
- DE-A1-102009 015 928
- DE-U1-202010 006 849
- US-A1- 2012 055 340

## Beschreibung

Die Erfindung betrifft eine Vorrichtung zur Beatmung eines Patienten, bei der ein Basisgerät zur Bereitstellung von Atemgas für die Patientenbeatmung sowie wenigstens eine Kontaktvorrichtung zur Verbindung mit wenigstens einem zu beatmenden Patienten verwendet wird, wobei die Kontaktvorrichtung mit dem Basisgerät derart gekoppelt ist, dass das Atemgas zwischen Basisgerät und Kontaktvorrichtung strömen kann, sowie bei der ein Atemgasadditiv in die Kontaktvorrichtung einspeisbar ist.

Die Erfindung betrifft darüber hinaus ein Verfahren zur Anreicherung von Atemgas mit Additiven, bei dem ein Basisgerät zur Bereitstellung von Atemgas verwendet wird.

Im Stand der Technik ist es bekannt, dass Vorrichtungen zur Beatmung eines Patienten verwendet werden, die über eine Funktion verfügen, die das dem Patienten zuzuführende Atemgas mit einer weiteren Komponente in additiver Weise anreichern.

Derartige Vorrichtungen folgen dem prinzipiellen Aufbau bestehend aus einem Basisgerät, einem an dem Basisgerät festgelegten Atemschlauch und einer wiederum an dem Atemschlauch festgelegten Kontaktvorrichtung, welche den Atemschlauch mit dem Patienten verbindet. Das Basisgerät verfügt dem Grunde nach über eine Ansaugvorrichtung zur Ansaugung von Umgebungsluft, die geräteintern gegebenenfalls als durch einen Filter gereinigtes Atemgas dem an dem Basisgerät festgelegten Atemschlauch zur Verfügung gestellt wird.

Dem Atemgas können vorrichtungsintern weitere, insbesondere gasförmige Komponenten, in additiver Weise beigemischt werden. Insbesondere wird häufig Sauerstoff dem Atemgas beigemischt, um Patienten in Notfallsituationen dieses Gas zuzuführen. Andere gasförmige Komponenten können zum Beispiel Distickstoffoxid (Lachgas) sein, das lange Zeit in der Medizin als Narkotikum verwendet wurde.

Die DE 20 2010 006 849 U1 offenbart eine Vorrichtung zur Beatmung eines Patienten mit einem als ein Beatmungsbeutel ausgebildeten Basisgerät, einer Kontaktvorrichtung und einer Additiveinspeisung in unmittelbarer Nähe zu der Kontaktvorrichtung.

In der DE 10 2007 05 8807 A1 wird bereits eine Vorrichtung zur Beatmung eines Patienten beschrieben, die mit einem Basisgerät und einer Kontaktvorrichtung versehen ist und bei der ein Atemgasadditiv in die Kontaktvorrichtung einspeisbar ist. Ähnliche Vorrichtungen werden auch in der US 2012/05 53 40 A1 sowie der DE 10 2004 045 693 A1 beschrieben.

Aus der DE 10 2009 015 928 A1 ist es bekannt, ein Atemgasadditiv mit einem vorgebbaren Volumenstrom und einem vorgebbaren Druck einzuspeisen.

Die DE 10 2009 015 928 A1 zeigt eine Vorrichtung zur Anreicherung von Atemgas mit Sauerstoff und offenbart ein entsprechendes Verfahren zur Anwendung der Vorrichtung für die Patientenbeatmung. Die Vorrichtung realisiert die Zuführung des Sauerstoffadditives in das vom Patienten einzuatmende Atemgas über ein Zuschaltventil sowie eine entsprechende Zuleitung in die Beatmungsvorrichtung. Das Zuschaltventil kann entweder innerhalb oder ausserhalb dieser bekannten Vorrichtung angeordnet sein. Die Vermischung erfolgt unabhängig von der internen oder externen Zuschaltventilanordnung immer vorrichtungsintern dadurch, dass der dosiert zugeführte Sauerstoff in die Atemgasstrecke beigemischt wird.

Innerhalb dieser Vorrichtung gemäß dem Stand der Technik ist eine Gebläsebox installiert, welche von dem Atemgas durchströmt wird und in die die Sauerstoffzuführung mündet, sodass an dieser Stelle in der Vorrichtung die Sauerstoffbeimischung erfolgt.

Neben dem Zuschaltventil ist weiterhin ein Rückschlagventil derart angeordnet erforderlich, dass bei entsprechenden Druckverhältnissen Atemgasdruck größer als der Sauerstoff-Zuführdruck das Rückschlagventil geschlossen ist und dadurch der Sauerstoffzuführschlauch nicht mit Atemgas geflutet werden kann.

Für die Dosierung des Sauerstoffes als Beimischungskomponente zum Atemgas wird ein Verfahren zur Steuerung des Zuschaltventils offenbart. Neben der Sauerstoffzuführung in eine geräteinterne Gebläsebox ist es ebenfalls bekannt, dass die Sauerstoffzuführung an einer Stelle des Beatmungsschlauches innerhalb der Vorrichtung erfolgt.

Nachteilig an diesen Vorrichtungen gemäß dem Stand der Technik ist, dass ein Zuschaltventil, üblicherweise als 2/2- oder 3/2-Wegeventil in Proportionalbauweise mit magnetischer Betätigung ausgestaltet verwendet werden muss. Dadurch steigen die Vorrichtungskosten. Weiterhin ist nachteilig, dass die Vorrichtung insbesondere bei der Notfallbeatmung kein mit hohen Anteilen von Sauerstoff angereichertes Atemgas zur Verfügung stellen kann. Ein weiterer gravierender Nachteil ist die Tatsache, dass sehr hohe Sauerstoffadditivvolumenströme erforderlich sind, um die Sauerstoffkonzentration innerhalb des anzureichernden Atemgases auf gewünschte Werte zu mischen.

Aufgabe der vorliegenden Erfindung ist es, eine Vorrichtung der einleitend genannten Art derart zu konstruieren, dass ein einfacher und kostengünstiger Aufbau unterstützt wird.

Diese Aufgabe wird erfindungsgemäß dadurch gelöst, dass eine Additiveinspeisung derart angeordnet ist, dass eine Zuführung des Atemgasadditives während eines Exspirationstaktes zur Flutung von Leitungen und Schläuchen des Basisgerätes sowie eines Atemschlauches vorgebbar ist, wobei die Zuführung des Atemgasadditives unmittelbar in den Kontaktvorrichtungen oder in einen der Kontaktvorrichtung benachbarten Atemschlauchabschnitt hinein erfolgt.

Es ist eine weitere Aufgabe der Erfindung, dem Patienten eine möglichst hohe Additivkonzentration als Atemluft zur Verfügung zu stellen.

Durch die Zuführung des Atemgasadditives unmittelbar in die Kontaktvorrichtung oder in einen der Kontaktvorrichtung benachbarten Atemschlauchabschnitt ist es möglich, die Additivkonzentration auch in Phasen großer Volumenströme bei annähernd 100% zu halten.

Zielgruppe der erfindungsgemäßen Vorrichtung und des Verfahrens zur Anreicherung von Atemgas mit Additiven sind vorzugsweise menschliche Patienten, die Lungenatmer sind. Die Arbeitsweise der Lunge ist taktend und somit diskontinuierlich. Ein Wechselspiel setzt sich zusammen aus Einatmen und Ausatmen. Bei dem Einatmungstakt wird Atemluft durch Expansion der Lunge angesaugt, bei dem Ausatmungstakt wird verwertete Atemluft als Abgas durch Kontraktion der Lunge ausgestoßen.

Einatmung und Ausatmung werden in der Medizin üblicherweise Inspiration und Exspiration genannt. Aufgrund dieser den Menschen und allen Säugetieren eigenen Arbeitsweise der Lunge muss auch ein Beatmungsgerät dieses Wechselspiel realisieren. Beatmungsgeräte werden prinzipiell dann angewendet, wenn die Lunge des Patienten das Wechselspiel nicht oder nicht ausreichend realisiert oder eine von der normalen Umgebungsluft, welche die Atemluft darstellt, abweichende Atemluftzusammensetzung erforderlich ist.

Eine Anwendung der Vorrichtung kann bei Patienten mit unterschiedlichen Krankheitsbildern an ventilatorischer Insuffizienz erfolgen. Typische Anwendungen sind obstruktive Ventilationsstörungen, z.B. COPD, Störungen der Atemmechanik, beispielsweise Skoliose, neuromuskuläre Erkrankungen, zentrale Atemregulationsstörungen oder obstruktives Schlafapnoesyndrom. Aufgrund der Möglichkeit, Additivquellen mit einer Volumenstromabgabemenge von 15 l/min einsetzen zu können, ist die Vorrichtung insbesondere als mobiles Notfallbeatmungsgerät geeignet. Aufgrund dieser Zusammenhänge ist die erfindungsgemäße Vorrichtung derart ausgestaltet, dass innerhalb eines Wechselspiels aus Inspiration und Exspiration zunächst ein Atemgasvolumenstrom von dem Basisgerät über den Atemschlauch bzw. Patientenschlauch und der Kontaktvorrichtung in die Lunge des zu beatmenden Patienten appliziert wird. Nach Abschluss der Inspiration wird durch ein innerhalb der Kontaktvorrichtung angeordnetes Patientenventil die Exspiration, d.h. die Exhalation des von der Lunge verwerteten Atemgases bewerkstelligt. Dazu ist das Patientenventil derart ausgebildet, dass ein entgegen der Atemgasvolumenstromrichtung bei der Inspiration gerichteter Volumenstrom des verwerteten Atemgases über das Patientenventil an die Umgebung abgegeben wird.

Infolge des Wechselspiels und der Funktion des Patientenventils, das von der Lunge verwertete Atemgas in die Umgebung und nicht in den Patientenschlauch zu leiten, alterniert der Volumenstrom innerhalb des Patientenschlauches und dem Basisgerät nicht. Der Atemgasvolumenstrom innerhalb der Vorrichtung ist somit bei Vorrichtungen gemäß dem Stand der Technik unidirektional und getaktet. Diesen Umstand macht sich die erfindungsgemäße Lehre zu eigen, um den Anteil des vorzugsweise gasförmigen Additives zum Atemgas erheblich zu erhöhen bis hin zu einer 100%igen Anreicherung.

Durch die kontinuierliche Zuführung des Atemgasadditives unmittelbar in die Kontaktvorrichtung oder in einen der Kontaktvorrichtung benachbarten Atemschlauchabschnitt und somit in den taktweise an die Patientenlunge abzugebenden Atemgasvolumenstrom ist es möglich, die Additivkonzentration während des Exspirationsvorgangs, bei dem der Atemgasvolumenstrom in idealisierter Betrachtung Null ist, in beliebiger Konzentration anzureichern oder vollständig zu ersetzen. Dies ist dadurch möglich, dass sowohl die Additivzuleitungen als auch der Patientenschlauch und die innerhalb des Basisgerätes vorhandenen Leitungen und Schläuche, welche aus Sicht der Additiveinspeisung vor dem druckerzeugenden Gebläse gelegen sind, mit dem Additiv teilweise oder vollständig geflutet werden können.

Während also bei einer Vorrichtung gemäß dem Stand der Technik das Additiv dem Atemgas während der Volumenstrombewegung beigemischt wird, nutzt die erfindungsgemäße Vorrichtung den durch den Exspirationsprozess verursachten Atemgas-Volumenstromstillstand, um das Atemgas teilweise oder vollständig in den Leitungen zu ersetzen und das ersetze Atemgas infolge entsprechenden Additivüberdruckes über eine Bypassleitung oder Gebläse-Kühlluftleitung auszustoßen.

Somit ist der Atemgasvolumenstrom nicht mehr nur unidirektional, sondern vielmehr bidirektional, es wird eine alternierende Atemgasvolumenstromrichtung innerhalb des Basisgerätes und dem Patientenschlauch realisiert. Mit dieser Konstruktion ist es nicht erforderlich, ein Ventil zum Zu- und Wegschalten des Sauerstoffadditives einzusetzen und die Erfindung unterstützt auf diese Weise eine kostengünstige Konstruktion. Ebenfalls positive Auswirkung auf die Kosten hat die Tatsache, dass gegenüber Geräten gemäß dem Stand der Technik nur eine Volumenstrommessung erfolgt.

Insbesondere bei Notfallbeatmungsgeräten ermöglicht die Erfindung erstmals auch die Bereitstellung von Sauerstoff durch Sauerstoffkonzentratoren. Notfallbeatmungsgeräte gemäß dem Stand der Technik sind, wie oben beschrieben, derart aufgebaut, dass die Additivzuführung durch Beimischung in den Atemgasvolumenstrom erfolgt. Deshalb sind Notfallbeatmungsgeräte auf mindestens 2,7 bar Versorgungsdruck angewiesen, um das Additiv in den Atemgasvolumenstrom einblasen zu können.

Sauerstoffkonzentratoren geben typischerweise Sauerstoff mit einem Druck zwischen 0,5 bar und 0,8 bar ab und sind somit nicht in der Lage, den Sauerstoff in den Atemgasvolumenstrom beizumischen. Hinzu tritt das Problem, dass Sauerstoffkonzentratoren einen zu geringen Sauerstoff-Spitzenvolumenstrom von bis zu maximal 15 l/min abgeben und Notfallbeatmungsgeräte Sauerstoff-Spitzenvolumenströme von bis zu 150 l/min benötigen, wenn sie den Sauerstoff in der konventionellen Weise beimischen. Mit dem erfindungsgemäßen Verfahren der Sauerstoffeinspeisung wird es jedoch möglich, auch Sauerstoffkonzentratoren als Sauerstoffadditivquelle zu verwenden.

In den Zeichnungen sind Ausführungsbeispiele der Erfindung schematisch dargestellt. Es zeigen:
- Fig. 1: in einer perspektivischen Darstellung den generellen Aufbau eines Beatmungsgerätes bestehend aus Basisgerät, Patienten- bzw. Atemschlauch und Kontaktvorrichtung,
- Fig. 2: einen erfindungsgemäßen Pneumatikplan in einer ersten Ausführungsform,
- Fig. 3: einen erfindungsgemäßen Pneumatikplan in einer zweiten Ausführungsform,
- Fig. 4: ein Diagramm, das über der Zeit Volumenströme und Drucksituationen darstellt,

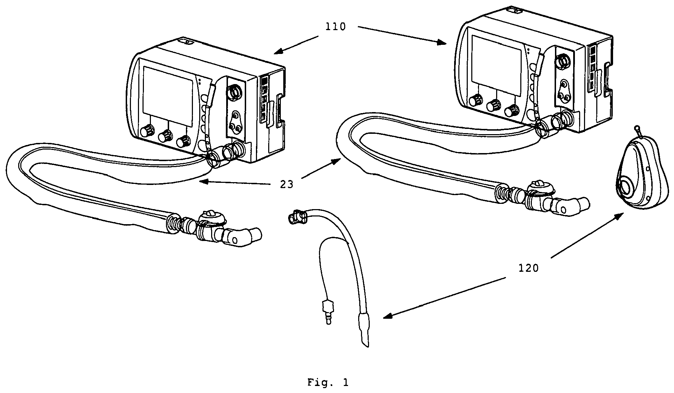

Fig. 1 zeigt den grundsätzlichen Aufbau einer Vorrichtung zur Beatmung und Anreicherung von Atemgas mit Additiven. An das Basisgerät (110) ist über einen als Kopplung ausgebildeten Patientenanschluss (17) der Patientenschlauch (23) anschließbar. Im Bereich einer dem Basisgerät (110) abgewandten Ausdehnung des Patientenschlauches (23) sind eine Kontaktvorrichtung (120) und ein Patientenventil (12) mit Additiveinspeisung (13) angeordnet. Es ist auch möglich, dass Kontaktvorrichtung (120) und Patientenventil (12) mit Additiveinspeisung (13) einteilig ausgeführt sind. Weiterhin ist es möglich, dass die Additiveinspeisung (13) als Zwischenstück des Patientenschlauches (23) ausgebildet ist.

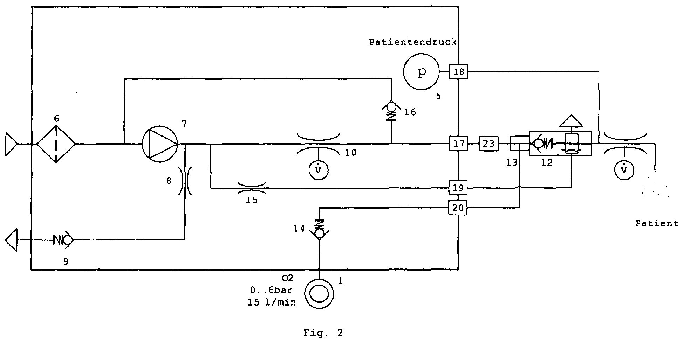

Fig. 2 zeigt den erfindungsgemäßen Pneumatikplan in einer ersten Ausführungsform. Die Zuführung des Atemgasadditives (13) erfolgt unmittelbar in die Kontaktvorrichtung (120) oder in einen der Kontaktvorrichtung benachbarten Atemschlauchabschnitt (23) hinein. Genauer gesagt erfolgt die Zuführung des Atemgasadditives (13) zwischen dem Schlauchsystem und dem Patientenventil.

Im Folgenden wird die Funktion exemplarisch anhand des Atemgasadditives Sauerstoff (O2) beschrieben. Andere, insbesondere gasförmige, Additive sind gleichfalls möglich, ggf. auch mehrere Additive einzeln oder als vermischte Additivrezeptur.

Das Basisgerät (110) verfügt über einen Inhalationsanschluss für die Zuführung von Sauerstoff, wie ihn z.B. einfache Druckminderer oder in Flaschen integrierte Druckminderer bieten. Auf diese Weise ist die sonst übliche Verwendung eines konventionellen High-Flow-O2-Druckeingangs und die Messung des FiO2 (der O2-Konzentration) mittels O2-Messzelle nicht erforderlich und unterstützt somit die einfache und kostensparende Gerätekonstruktion.

Infolge des Erfindungsgedankens, dass das Atemgasadditiv - hier beispielhaft Sauerstoff - während des Exspirationstaktes zur Flutung der Leitungen und Schläuche des Basisgerätes (110) und Atemschlauches (23) genutzt wird, ist eine Steuerung und Messung der O2-Beimischung nicht erforderlich. Der Inhalationsanschluss liefert einen mittleren, einstellbaren O2-Volumenstrom von bis zu 15 l/min. Im Mittel ist dies ein ausreichend großer Volumenstrom für die Versorgung der meisten Patienten im Hinblick auf einen normalen, durchschnittlichen Sauerstoffbedarf.

Es gibt aber auch Phasen der Inspiration, in denen der tatsächlich inhalierte Volumenstrom so groß wird, dass er über diesen Wert hinausgeht. Als Folge fällt die O2-Konzentration bei einer klassischen Inhalation stark ab, wenn ein Beatmungsgerät mit Sauerstoffbeimischung gemäß dem Stand der Technik eingesetzt wird.

Durch die Zuführung des Sauerstoffadditives unmittelbar in die Kontaktvorrichtung (120), oder in einen der Kontaktvorrichtung benachbarten Atemschlauchabschnitt (23), ist es möglich, die Sauerstoffkonzentration je nach eingestellten Volumenstromverhältnissen in beliebiger Konzentration anzureichern oder vollständig zu ersetzen. Dies ist dadurch möglich, dass sowohl die Sauerstoffzuleitung, welche mit einem Rückschlagventil (14) ausgestattet ist, als auch der Patientenschlauch (23) und die innerhalb des Basisgerätes vorhandenen Leitungen und Schläuche, welche aus Sicht der Additiveinspeisung (13) vor dem druckerzeugenden Gebläse (7) gelegen sind mit dem Sauerstoff teilweise oder vollständig geflutet werden.

Praktisch werden also die Sauerstoffzuleitung, Patientenschlauch (23) und die innerhalb des Basisgerätes (110) vorhandenen Leitungen und Schläuche als Sauerstoff-Reservoire genutzt und in der Exspirationsphase mit Sauerstoff teilweise oder vollständig gefüllt. Dies geschieht dadurch, dass durch den O2-Anschluss (1) der Sauerstoff mit einem Druck von vorzugsweise größer Null bis 6 bar bereitgestellt wird.

Wird entweder der Druck des Gebläses (7) oder des Rückschlagventils (9) unterhalb des Einspeisungsdruckes des Sauerstoffes eingestellt, kann Sauerstoff durch die Sauerstoffzuleitung in den Patientenschlauch (23) und die innerhalb des Basisgerätes vorhandenen Leitungen und Schläuche, welche aus Sicht der Additiveinspeisung (13) vor dem druckerzeugenden Gebläse (7) gelegen sind einströmen und verdrängt über die Leitung und Rückschlagventil (9) das ersetzte Atemgas in die Umgebung.

Praktisch wird also durch die entstehende Sauerstoffsäule das Atemgas teilweise oder vollständig aus den oben genannten Bauelementen verdrängt. Auf diese Weise kann durch die Abstimmung der Druckverhältnisse von Sauerstoffeinspeisungsdruck und Atemgasdruck (durch Gebläsedruck und, oder eingestelltem Druck an dem Rückschlagventil (9)), sowie den durch die Volumina der Sauerstoffzuleitung, des Patientenschlauches (23) und die innerhalb des Basisgerätes vorhandenen Leitungen und Schläuche eine teilweise oder vollständige Befüllung mit Sauerstoff erreicht werden. Dies ermöglicht eine O2-Konzentration auch in Phasen großer Volumenströme von bis zu 100%.

In Richtung der Atemgas-Volumenstromrichtung hinter der Additiveinspeisung (13), d.h. in Richtung Patientenlunge, ist als Bestandteil der Kontaktvorrichtung (120) und bevorzugt innerhalb des Patientenventils (12) ein Rückschlagventil vorgesehen, sodass bei der Exspiration keine Rückatmung verwerteten und somit sauerstoffarmem Atemgases in die Vorrichtung (100) möglich ist und somit keine Reduzierung des Sauerstoffanteils in dem Patientenschlauch (23) und die innerhalb des Basisgerätes (110) vorhandenen Leitungen und Schläuche erfolgt.

Die Schlauch - und Leitungsvolumina des Patientenschlauches (23) und die innerhalb des Basisgerätes (110) vorhandenen Leitungen und Schläuche sind derart abgestimmt, dass ein Tidalvolumen (Atemzugvolumen) von bis zu 600ml mit bis zu 100% Sauerstoffkonzentration pro Inspirationstakt verabreicht werden kann.

Der Volumenstromsensor, der nach der Kontaktvorrichtung (120) und vor der schematisch dargestellten Patientenlunge eingezeichnet ist, ist nicht Bestandteil der Vorrichtung.

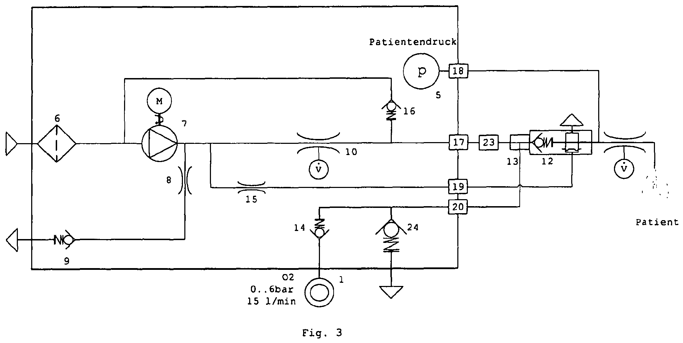

Fig. 3 zeigt den erfindungsgemäßen Pneumatikplan in einer weiteren Ausführungsform. Im Vergleich zum Pneumatikplan der ersten Ausführungsform in Fig. 2 wird hier eine Sauerstoffquelle verwendet, die bei einem möglichen Druck von bis zu 6 bar einen Sauerstoffvolumenstrom von 15 l/min abgeben kann.

Die Zuleitung des Sauerstoffadditives über das Rückschlagventil (14) innerhalb des Basisgerätes (110) zum O2-Anschlussstutzen (20) weist eine zusätzliche, in die Umgebung mündende Entlastungsleitung auf, die mit einem Rückschlagventil (24) ausgestattet ist. Das Rückschlagventil (24) arbeitet als Sicherheitsventil mit einem eingestellten Druck von 100 hPa. Das bedeutet, dass das geschlossene System bestehend aus den Komponenten Rückschlagventil (14), O2-Stutzen (20), O2-Einspeisung (13), Patientenschlauch (23), Patientenanschluss (17), Ruckschlagventil (16), Volumenstromsensor (10), Gebläse (7) und Kühlblende (8) sowohl über das Rückschlagventil (9) als auch über das Sicherheitsventil (24) entlastet werden kann.

Der Vorteil liegt in der genauen Druckbegrenzung innerhalb des geschlossenen Systems und damit dem Zuführdruck des Atemluft-Sauerstoffgemisches generell sowie der möglicherweise auftretenden Druckspitze im Moment des Öffnens des Patientenventils (12) zu Beginn der Inspirationsphase. Sollte eine Druckspitze am Patientenventil (12) auftreten, so hätte das Rückschlagventil (24) hierauf keinen Einfluss. Allein der Druck beziehungsweise Flow aus dem Bauteil (1) wird wirksam reduziert.

Das Sicherheitsventil (24) begrenzt den Systemdruck auf den eingestellten IPAP-Druck (Inspiratory Positive Airway Pressure - positiver inspiratorischer Atemwegsdruck).

Es sind weiterhin Temperatursensoren (7.1, 7.2, 10.1) vorgesehen, die eine Anpassung an konkret vorliegende Temperaturen unterstützen. Insbesondere kann eine zu geringe Kühlung aufgrund zu geringer Volumenströme erfasst und einer derartigen zu geringen Kühlung entgegengewirkt werden.

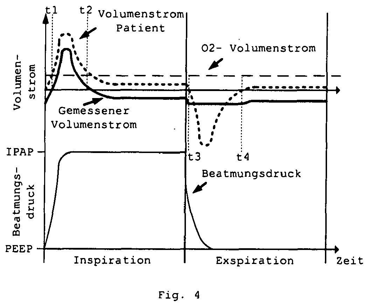

Fig. 4 zeigt ein Diagramm, das über der Zeit Volumenströme und Drucksituationen darstellt. Illustriert wird das Verhalten während der Inspiration und der Exspiration. Während der Inspiration ist der Druck des Gebläses (7) höher als der Druck in der Lunge des Patienten und es fließt ein Patienten-Volumenstrom (fett-gestrichelte Linie im Zeit-Volumenstromdiagrammteil) in Richtung Patient durch das innerhalb des Patientenventils (12) angeordnete Rückschlagventil.

Dieser Volumenstrom setzt sich aus dem konstanten O2-Volumenstrom (schmalgestrichelte Linie im Zeit-Volumenstromdiagrammteil) und dem Volumenstrom bestehend aus Atemgas und Sauerstoff zusammen. Der Atemgas-Sauerstoff-Volumenstrom wird an dem Volumenstromsensor (10, 11) gemessen (durchgezogenen Linie im Zeit-Volumenstromdiagrammteil - gemessener Volumenstrom), der Sauerstoffvolumenstrom ist voreingestellt.

Solange der Volumenstrom durch das innerhalb des Patientenventils (12) angeordnete Rückschlagventil in Richtung Patientenlunge kleiner dem zugeführten O2-Volumenstrom ist, fließt der nicht applizierte Teil des O2-Volumenstroms in Richtung Beatmungsgerät. Das Zeit-Volumenstromdiagramm stellt diese Situation durch die Kennlinien im Bereich von t=0 bis t=t1 dar.

Sobald der Volumenstrom durch das innerhalb des Patientenventils (12) angeordnete Rückschlagventil in Richtung Patientenlunge größer dem zugeführten O2-Volumenstrom ist, fließt ein zusätzlicher Volumenstrom bestehend aus Atemgas und Sauerstoff aus Richtung des Gebläses (7) aus dem Basisgerät (110) und über die Volumenstrommessung (10, 11) durch den Patientenschlauch (23) zur Kontaktvorrichtung (120) und weiter zur Patientenlunge.

Nach dem Anschwellen des Patienten-Volumenstromes füllt sich die Lunge mit Beatmungsgas und der Volumenstrom sinkt wieder ab. Das Zeit-Volumenstromdiagramm stellt diese Situation durch die Kennlinien im Bereich von t=t1 bis t=t2 dar. Sobald der Patienten-Volumenstrom kleiner ist als der Sauerstoffvolumenstrom, fließt wieder Sauerstoff in das Sauerstoff-Reservoir des Basisgerätes, bestehend aus den innerhalb des Basisgerätes vorhandenen Leitungen und Schläuchen sowie dem Patientenschlauch (23), d.h. auch durch die Volumenstrommessung (10, 11) entgegen der Atemgasvolumenstromrichtung. Das Zeit-Volumenstromdiagramm stellt diese Situation durch die Kennlinien im Bereich von t=t2 bis t=t3 dar.

Wäre das System in Richtung Patientenlunge leckagefrei, könnte bereits in diesem Moment der tatsächlich eingespeiste O2-Volumenstrom an dem Volumenstromsensor (10, 11) gemessen werden. Dies ist in der Realität keine zulässige Annahme, da die Kontaktvorrichtung (120), welche an dem Patienten angelegt werden muss, nicht vollständig dichtet.

Erst wenn der Beatmungsdruck durch das Basisgerät (110) infolge einer entsprechenden Steuerung des Gebläses (7) beziehungsweise des Antriebsmotors M gemäß dem Zeit-Beatmungsdruckdiagramm abfällt und sich dadurch bedingt die Druckverhältnisse am im Patientenventil (12) angeordneten Rückschlagventil umkehren und sich das Rückschlagventil schließt, ist eine mögliche Leckage entkoppelt. Dann kann der aktuelle O2-Volumenstrom gemessen werden.

Das Zeit-Volumenstromdiagramm stellt diese Situation durch die Kennlinien im Bereich von t=t3 bis t=t4 dar. Unter der hier vorliegenden Bedingung, dass der O2-Volumenstrom konstant ist, lässt sich sowohl die mittlere O2-Konzentration, als auch der tatsächlich abgegebene Patienten-Volumenstrom bestimmen durch einfache Differenzwertbildung.

Wird der Sauerstoff-Volumenstrom an der Sauerstoffquelle auf einen anderen konstanten Wert eingestellt ändert sich die Sauerstoffkonzentration in dem Atemgas. Das bedeutet, dass durch den O2-Volumenstrom die Sauerstoffkonzentration einstellbar ist.

Das Zeit-Beatmungsdruckdiagramm zeigt für die Phasen der Inspiration und Exspiration den von der Vorrichtung realisierten Beatmungsdruck auf. Der Beatmungsdruck kann dabei Werte zwischen einem Minimalwert und einem Maximalwert annehmen. Der Minimalwert entspricht dem PEEP, was für "Positive End Expiratory Pressure" oder "positiv-endexpiratorischer Druck" steht. Der PEEP ist der geringste Druckwert in der Lunge am Ende der Exspiration. Der Druck wird genau genommen exspiratorisch zwar nicht kleiner als der PEEP, die Bedeutung des PEEP ergibt sich aber daraus, dass sich endinspiratorisch dieser Beatmungsdruck einstellen sollte.

Der PEEP wird als Druckwert größer Null an der Vorrichtung eingestellt durch eine PEEP-Steuerblende (15), die mittels eines PEEP-Stutzens (19) mit dem Patientenventil (12) verbunden ist mit dem Ziel, die Lunge am Ende eines Atemzyklus offenzuhalten und der Neigung von beatmeten Patienten zum Kollaps entgegenzuwirken. Der Maximalwert, den der Beatmungsdruck annehmen kann, ist der IPAP (Inspiratory Positive Airway Pressure - positiver inspiratorischer Atemwegsdruck). Die Druckverhältnisse werden durch einen Atemwegdrucksensor (5) aufgenommen, der innerhalb des Basisgerätes (110) angeordnet und über einen PAW-Stutzen (18) mit der Kontaktvorrichtung (120) verbunden ist.

Gemäß einer weiteren bevorzugten Ausführungsform ist auch darauf hinzuweisen, dass die Vorrichtung eine Kühlung des Gebläses ermöglicht. Dies kann beispielsweise entsprechend den Erläuterungen in Fig. 3, insbesondere unter Berücksichtigung der Bauteile (8 und 9) erfolgen. Das Kühlprinzip beruht insbesondere auch darauf, dass sich das Gebläse selbst über eine feste oder verstellbare Blende kühlt.

Wie bereits erwähnt wird von der Atemgasadditivquelle ein konstanter Additivvolumenstrom von mit zu 15 l/min bereitgestellt. Besonders bevorzugt ist es, dass die Atemgasadditivquelle einen Druck von etwa 100 hPA bereitstellt.

## Patentansprüche

1. Vorrichtung zur Beatmung eines Patienten, aufweisend ein Basisgerät (110) zur Bereitstellung von Atemgas für die Patientenbeatmung sowie wenigstens eine Kontaktvorrichtung (120) zur Verbindung mit wenigstens einem zu beatmenden Patienten und ein Patientenventil (12), wobei die Kontaktvorrichtung (120) mit dem Basisgerät (110) derart gekoppelt ist, dass das Atemgas zwischen Basisgerät (110) und Kontaktvorrichtung (120) strömen kann, sowie bei der ein Atemgasadditiv mithilfe einer Additiveinspeisung (13) in die Kontaktvorrichtung (120) einspeisbar ist , **dadurch gekennzeichnet, dass** die Additiveinspeisung (13) in Strömungsrichtung des Atemgases während eines Inspirationstakts vor dem Patientenventil (12) angeordnet ist, sodass eine Zuführung des Atemgasadditives während eines Exspirationstaktes zur Flutung von Leitungen und Schläuchen des Basisgerätes (110) sowie eines Atemschlauches (23) vorgebbar ist, wobei die Additiveinspeisung (13) unmittelbar in die Kontaktvorrichtung (120) oder in einen der Kontaktvorrichtung (120) benachbarten Atemschlauchabschnitt mündet.

2. Vorrichtung nach Anspruch 1, **dadurch gekennzeichnet, dass** die Kontaktvorrichtung (120) das Patientenventil (12) und die Additiveinspeisung (13) aufweist.

3. Vorrichtung nach Anspruch 2, **dadurch gekennzeichnet, dass** das Patientenventil (12) und die Additiveinspeisung (13) innerhalb der Kontaktvorrichtung (120) einteilig ausgeführt sind.

4. Vorrichtung nach einem der Ansprüche 1 bis 3, **dadurch gekennzeichnet, dass** die Vorrichtung zur Unterstützung einer Atemgasadditivbeimischung zum Atemgas ausgebildet ist.

5. Vorrichtung nach einem der Ansprüche 1 bis 3, **dadurch gekennzeichnet, dass** die Vorrichtung zur Unterstützung einer Atemgasadditivkonzentration in dem Atemgas von bis zu 100% ausgebildet ist.

6. Vorrichtung nach einem der vorgehenden Ansprüche, **dadurch gekennzeichnet, dass** die Atemgasadditivzuleitung, der Patientenschlauch (23) und die innerhalb des Basisgerätes (110) vorhandenen Leitungen und Schläuche als geschlossene Atemgasadditiv-Reservoire nutzbar sind.

7. Vorrichtung nach einem der vorgehenden Ansprüche, **dadurch gekennzeichnet, dass** die Vorrichtung für die Bereitstellung des Atemgasadditivs durch eine an die Vorrichtung koppelbaren Atemgasadditivquelle (1) ausgebildet ist.

8. Vorrichtung nach Anspruch 7, **dadurch gekennzeichnet dass** sie zur Realisierung eines konstanten Additivvolumenstromes innerhalb eines Druckniveaus im Bereich von 0 bis 6 bar von der Atemgasadditivquelle (1) ausgebildet ist.

9. Vorrichtung nach Anspruch 7, **dadurch gekennzeichnet, dass** sie zur Bereitstellung eines konstanten Additivvolumenstromes von bis zu 15l/min von der Atemgasadditivquelle ausgebildet ist.

10. Vorrichtung nach Anspruch 6, **dadurch gekennzeichnet, dass** die Atemgasadditivzuleitung durch einen Abschnitt innerhalb des Basisgerätes (110) und einen Abschnitt außerhalb des Basisgerätes (110) gebildet ist.

11. Vorrichtung nach Anspruch 6, **dadurch gekennzeichnet, dass** die Atemgasadditivzuleitung ein Rückschlagventil (14) aufweist, das derart orientiert ist, dass eine Strömung in Richtung der Atemgasadditivquelle gesperrt ist.

12. Vorrichtung nach Anspruch 6 **dadurch gekennzeichnet dass** sie eine Druckbegrenzungsvorrichtung zur Entlastung des geschlossenen Atemgasadditiv-Reservoirs aufweist.

13. Vorrichtung nach Anspruch 12, **dadurch gekennzeichnet, dass** die Druckbegrenzungsvorrichtung zur Entlastung des geschlossenen Atemgasadditiv-Reservoirs durch eine an die Atemgasadditivzuleitung angeschlossene zusätzliche, in die Umgebung mündende, Entlastungsleitung mit einem Sicherheitsventil (24) gebildet ist.

14. Vorrichtung nach Anspruch 1, **dadurch gekennzeichnet, dass** eine Volumenstrommessung (10, 11) zur Bestimmung des Atemgasvolumenstromes und zur Atemgasadditivkonzentration verwendet ist.

15. Vorrichtung nach Anspruch 1, **dadurch gekennzeichnet, dass** die Vorrichtung eine mobile Notfallbeatmungsvorrichtung ist.

16. Vorrichtung nach Anspruch 1, **dadurch gekennzeichnet, dass** das Atemgasadditiv gasförmig ist.

17. Vorrichtung nach Anspruch 1, **dadurch gekennzeichnet, dass** das Atemgasadditiv Sauerstoff ist.

18. Verfahren zur Anreicherung von Atemgas mit Additiven, aufweisend die Schritte
a) Bereitstellung an einer Kontaktvorrichtung (120) eines druckbeaufschlagten Atemgasvolumenstromes durch eine Vorrichtung gemäß einem der Ansprüche 1 bis 17,
b) Bereitstellung eines druckbeaufschlagten Atemgasadditiv-Volumenstromes,
**dadurch gekennzeichnet, dass** die Bereitstellung des druckbeaufschlagten Atemgasvolumenstroms sowie Atemgasadditiv-Volumenstroms an der Kontaktvorrichtung (120) derart erfolgt, dass während des Exspirationstakts der Atemgasadditiv-Volumenstrom wenigstens teilweise in das zumindest durch Bereiche des Atemschlauches (23) realisierte Additiv-Reservoir strömt, sodass eine Anreicherung des Atemgasadditives in dem im Additiv-Reservoir befindlichen Atemgas erfolgt.

19. Verfahren gemäß Anspruch 18, **dadurch gekennzeichnet, dass** der an der Kontaktvorrichtung (120) zur Abgabe bereitgestellte Anteil des Atemgasadditiv-Volumenstroms bis zu 100% des gesamten Atemgas-Volumenstromes beträgt.

20. Verfahren gemäß Anspruch 18, **dadurch gekennzeichnet, dass** der Schritt b) durch einen einstellbaren konstanten Atemgasadditiv-Volumenstrom erfolgt.

21. Verfahren gemäß Anspruch 18. **dadurch gekennzeichnet, dass** durch eine Volumenstrommessung (10, 11) sowohl die mittlere Atemgasadditivkonzentration als auch der tatsächlich abgegebene Atemgas-Volumenstrom bestimmbar sind.

22. Verfahren gemäß Anspruch18, **dadurch gekennzeichnet, dass** durch die Einstellung des konstanten Atemgasadditiv-Volumenstroms die AtemgasadditivKonzentration in dem Atemgas-Volumenstrom änderbar ist.

## Claims

1. Device for ventilating a patient, having a base unit (110) for providing respiratory gas for the patient ventilation, and at least one contact device (120) for connection to at least one patient to be ventilated, and a patient valve (12), wherein the contact device (120) is coupled to the base unit (110) in such a way that the respiratory gas can flow between base unit (110) and contact device (120), and in which a respiratory gas additive is feedable into the contact device (120) with the aid of an additive feed (13), **characterized in that** the additive feed (13) is arranged upstream from the patient valve (12) in the direction of flow of the respiratory gas during an inhalation cycle, such that it is possible to predefine a delivery of the respiratory gas additive during an exhalation cycle for flooding lines and tubes of the base unit (110) and a respiratory tube (23), wherein the additive feed (13) opens directly into the contact device (120) or into a respiratory tube section adjacent to the contact device (120).

2. Device according to Claim 1, **characterized in that** the contact device (120) has the patient valve (12) and the additive feed (13).

3. Device according to Claim 2, **characterized in that** the patient valve (12) and the additive feed (13) are formed in one piece inside the contact device (120) .

4. Device according to one of Claims 1 to 3, **characterized in that** the device is designed to support an admixture of respiratory gas additive to the respiratory gas.

5. Device according to one of Claims 1 to 3, **characterized in that** the device is designed to support a respiratory gas additive concentration in the respiratory gas of up to 100%.

6. Device according to one of the preceding claims, **characterized in that** the respiratory gas additive feed line, the patient tube (23) and the lines and tubes present inside the base unit (110) are usable as closed respiratory gas additive reservoirs.

7. Device according to one of the preceding claims, **characterized in that** the device is designed to provide the respiratory gas additive from a respiratory gas additive source (1) that can be coupled to the device.

8. Device according to Claim 7, **characterized in that** it is designed to provide a constant additive volume flow within a pressure level in the range from 0 to 6 bar from the respiratory gas additive source (1).

9. Device according to Claim 7, **characterized in that** it is designed to provide a constant additive volume flow of up to 15 l/min from the respiratory gas additive source.

10. Device according to Claim 6, **characterized in that** the respiratory gas additive feed line is formed by a section inside the base unit (110) and a section outside the base unit (110).

11. Device according to Claim 6, **characterized in that** the respiratory gas additive feed line has a check valve (14), which is oriented in such a way that a flow in the direction of the respiratory gas additive source is blocked.

12. Device according to Claim 6, **characterized in that** it has a pressure-limiting device for relieving the closed respiratory gas additive reservoir.

13. Device according to Claim 12, **characterized in that** the pressure-limiting device for relieving the closed respiratory gas additive reservoir is formed by an additional relief line with a safety valve (24), which additional relief line is attached to the respiratory gas additive feed line and opens into the surroundings.

14. Device according to Claim 1, **characterized in that** a volume flow measurement (10, 11) is used for determining the respiratory gas volume flow and for the respiratory gas additive concentration.

15. Device according to Claim 1, **characterized in that** the device is a mobile emergency ventilation device.

16. Device according to Claim 1, **characterized in that** the respiratory gas additive is gaseous.

17. Device according to Claim 1, **characterized in that** the respiratory gas additive is oxygen.

18. Method for enriching respiratory gas with additives, having the steps of
a) providing, at a contact device (120), a pressurized respiratory gas volume flow by means of a device according to one of Claims 1 to 17,
b) providing a pressurized respiratory gas additive volume flow,
**characterized in that** the provision of the pressurized respiratory gas volume flow and respiratory gas additive volume flow at the contact device (120) is effected in such a way that, during the exhalation cycle, the respiratory gas additive volume flow at least partially flows into the additive reservoir realized at least by regions of the respiratory tube (23), such that an enrichment of the respiratory gas additive in the respiratory gas located in the additive reservoir takes place.

19. Method according to Claim 18, **characterized in that** the portion of the respiratory gas additive volume flow that is provided for delivery at the contact device (120) constitutes up to 100% of the total respiratory gas volume flow.

20. Method according to Claim 18, **characterized in that** step b) is effected by an adjustable constant respiratory gas additive volume flow.

21. Method according to Claim 18, **characterized in that** the mean respiratory gas additive concentration and also the actually delivered respiratory gas volume flow can be determined by a volume flow measurement (10, 11).

22. Method according to Claim 18, **characterized in that** the respiratory gas additive concentration in the respiratory gas volume flow is modifiable by the adjustment of the constant respiratory gas additive volume flow.

## Revendications

1. Dispositif de ventilation d'un patient, présentant un appareil de base (110) pour la fourniture de gaz respiratoire pour la ventilation du patient ainsi qu'au moins un dispositif de contact (120) pour la liaison avec au moins un patient à ventiler et une soupape de patient (12), dans lequel le dispositif de contact (120) est couplé à l'appareil de base (110) de telle manière que le gaz respiratoire puisse s'écouler entre l'appareil de base (110) et le dispositif de contact (120), et dans lequel un additif de gaz respiratoire peut être fourni dans le dispositif de contact (120) à l'aide d'une alimentation d'additif (13), **caractérisé en ce que** l'alimentation d'additif (13) est disposée dans la direction d'écoulement du gaz respiratoire pendant une phase d'inspiration avant la soupape de patient (12), de telle manière qu'un apport de l'additif de gaz respiratoire pendant une phase d'expiration puisse être prédéterminé pour le remplissage de conduites et de tuyaux flexibles de l'appareil de base (110) ainsi que d'un tuyau flexible respiratoire (23), dans lequel l'alimentation d'additif (13) débouche directement dans le dispositif de contact (120) ou dans une partie de tuyau flexible respiratoire proche du dispositif de contact (120).

2. Dispositif selon la revendication 1, **caractérisé en ce que** le dispositif de contact (120) présente la soupape de patient (12) et l'alimentation d'additif (13).

3. Dispositif selon la revendication 2, **caractérisé en ce que** la soupape de patient (12) et l'alimentation d'additif (13) sont réalisées en une seule pièce à l'intérieur du dispositif de contact (120).

4. Dispositif selon l'une quelconque des revendications 1 à 3, **caractérisé en ce que** le dispositif est configuré pour soutenir un mélange d'additif de gaz respiratoire dans le gaz respiratoire.

5. Dispositif selon l'une quelconque des revendications 1 à 3, **caractérisé en ce que** le dispositif est configuré pour assurer une concentration d'additif de gaz respiratoire dans le gaz respiratoire jusqu'à 100 %.

6. Dispositif selon l'une quelconque des revendications précédentes, **caractérisé en ce que** la conduite d'arrivée d'additif de gaz respiratoire, le tuyau souple de patient (23) et les conduites et les tuyaux flexibles se trouvant à l'intérieur de l'appareil de base (110) peuvent être utilisés comme réservoirs d'additif de gaz respiratoire fermés.

7. Dispositif selon l'une quelconque des revendications précédentes, **caractérisé en ce que** le dispositif est configuré pour fournir l'additif de gaz respiratoire par une source d'additif de gaz respiratoire (1) pouvant être couplée au dispositif.

8. Dispositif selon la revendication 7, **caractérisé en ce qu'**il est configuré pour la réalisation d'un courant volumique d'additif constant à l'intérieur d'un niveau de pression dans la plage de 0 à 6 bars à partir de la source d'additif de gaz respiratoire (1).

9. Dispositif selon la revendication 7, **caractérisé en ce qu'**il est configuré pour la fourniture d'un courant volumique d'additif constant allant jusqu'à 15 l/min à partir de la source d'additif de gaz respiratoire.

10. Dispositif selon la revendication 6, **caractérisé en ce que** la conduite d'arrivée d'additif de gaz respiratoire est formée par une partie à l'intérieur de l'appareil de base (110) et une partie à l'extérieur de l'appareil de base (110).

11. Dispositif selon la revendication 6, **caractérisé en ce que** la conduite d'arrivée d'additif de gaz respiratoire présente un clapet anti-retour (14), qui est orienté de telle manière qu'un écoulement en direction de la source d'additif de gaz respiratoire soit bloqué.

12. Dispositif selon la revendication 6, **caractérisé en ce qu'**il présente un dispositif de limitation de pression pour le délestage du réservoir d'additif de gaz respiratoire fermé.

13. Dispositif selon la revendication 12, **caractérisé en ce que** le dispositif de limitation de pression pour le délestage du réservoir d'additif de gaz respiratoire fermé est formé par une conduite de délestage raccordée à la conduite d'arrivée d'additif de gaz respiratoire et débouchant dans l'atmosphère, avec une soupape de sécurité (24).

14. Dispositif selon la revendication 1, **caractérisé en ce qu'**une mesure de courant volumique (10, 11) est utilisée pour la détermination du courant volumique de gaz respiratoire et pour la concentration d'additif de gaz respiratoire.

15. Dispositif selon la revendication 1, **caractérisé en ce que** le dispositif est un dispositif de ventilation d'urgence mobile.

16. Dispositif selon la revendication 1, **caractérisé en ce que** l'additif de gaz respiratoire est gazeux.

17. Dispositif selon la revendication 1, **caractérisé en ce que** l'additif de gaz respiratoire est l'oxygène.

18. Procédé d'enrichissement de gaz respiratoire par des additifs, présentant les étapes suivantes:
a) fourniture à un dispositif de contact (120) d'un courant volumique de gaz respiratoire sous pression par un dispositif selon l'une quelconque des revendications 1 à 17,
b) fourniture d'un courant volumique d'additif de gaz respiratoire sous pression,
**caractérisé en ce que** la fourniture du courant volumique de gaz respiratoire sous pression ainsi que du courant volumique d'additif de gaz respiratoire au dispositif de contact (120) est effectuée de telle manière que pendant la phase d'expiration le courant volumique d'additif de gaz respiratoire s'écoule au moins en partie dans le réservoir d'additif réalisé au moins par des parties du tuyau flexible respiratoire (23), de telle manière qu'il se produise un enrichissement de l'additif de gaz respiratoire dans le gaz respiratoire se trouvant dans le réservoir d'additif.

19. Procédé selon la revendication 18, **caractérisé en ce que** la part du courant volumique d'additif de gaz respiratoire préparée pour être fournie au dispositif de contact (120) vaut jusqu'à 100 % du courant volumique de gaz respiratoire total.

20. Procédé selon la revendication 18, **caractérisé en ce que** l'étape b) est effectuée par un courant volumique d'additif de gaz respiratoire constant réglable.

21. Procédé selon la revendication 18, **caractérisé en ce qu'**aussi bien la concentration moyenne d'additif de gaz respiratoire que le courant volumique de gaz respiratoire effectivement fourni peuvent être déterminés par une mesure de courant volumique (10, 11).

22. Procédé selon la revendication 18, **caractérisé en ce que** la concentration d'additif de gaz respiratoire dans le courant volumique de gaz respiratoire peut être modifiée par le réglage du courant volumique constant d'additif de gaz respiratoire.
